# EUROPEAN PATENT SPECIFICATION

(11) **EP 2 062 946 B1**
(45) Date of publication and mention of the grant of the patent: **06.06.2012**
(21) Application number: 07114266.5
(22) Date of filing: 13.08.2007
(51) Int. Cl.: C09B 67/08, A61K 8/11, A61K 9/16, A61K 9/51, C09B 67/02

(54) **Dye-loaded particles**
Farbstoffhaltige Teilchen
Particules chargées de colorant

(43) Date of publication of application: 27.05.2009
(73) Proprietor: Procter & Gamble International Operations SA, 1213 Geneva (CH)
(72) Inventor: Alberius, Peter, Carl, Anders, 183 51, Taby (SE); Corkery, Robert, William, 11727, Stockholm (SE)
(74) Representative: Simpson, Kirsty Mairi

(56) References cited:
- WO-A-00/09652
- WO-A-2004/081222
- DATABASE WPI Week 200359 Thomson Scientific, London, GB; AN 2003-627380 XP002493028 & WO 03/060037 A (TECHNO NETWORK SHIKOKU CO LTD) 24 July 2003 (2003-07-24)
- MAMORU AIZAWA ET AL: "Preparation of Spherical Hydrous Silica Oxide Particles under Acidic Condition via Sol-Gel Processing" 1 December 2000 (2000-12-01), JOURNAL OF SOL-GEL SCIENCE AND TECHNOLOGY, KLUWER ACADEMIC PUBLISHERS, BO, PAGE(S) 329 - 332 , XP019212659 ISSN: 1573-4846 * paragraph [02.1] * * paragraph [02.2] * * figure 6 *

## Description

The present invention relates to encapsulated dyes.

### BACKGROUND TO THE INVENTION

The provision of dyes is key in many fields of technology, for instance in the preparation of cosmetic, personal care and health compositions, detergent compositions and in printing technologies, to name but a few.

Particles incorporating dyes for use in these fields arc described in WO-A-2004/081222. This document describes a process for manufacturing encapsulated dyes using the well-known sol-gel methodology, which is an emulsion technique resulting in a core/shell structure (a core of dye surrounded by a shell of a material, such as silica). Further examples of particles of the art are found in US-A-2005/0276774 and US-A-2005/0265938, which describe the production of such particles by dispersive techniques and micelle formation respectively. However, particles known in the art often exhibit leakage of the dyes from the particles into which they have been incorporated, which is clearly undesirable. WO 03/060037, WO 00/09652 and Mamoru Aizawa et al., Journal of Sol-Gel Science and Technology, Vol. 19, pages 329-332, 2000, also discuss methods for the siliceous encapsulation of dyes.

Accordingly, there is a need for new dye particles, which can be reliably and effectively incorporated into a wide variety of end compositions whilst retraining all of the benefits of dyes already known in the art, i.e. good chemical and physical stability, colour fastness and tint strength as, well as an acceptable environmental profile, but which at the same time show negligible to no leakage of the dyes from the particles over their lifetime.

### SUMMARY OF THE INVENTION

According to the invention, an amorphous particle is provided comprising a homogeneous distribution of one or more dyes encapsulated by an amorphous, siliceous encapsulating agent, wherein the amorphous particle comprises from 3% to 20% dye, by weight of the particle, and wherein the dye is homogeneously dispersed throughout the particle.

### BRIEF DESCRIPTION OF THE DRAWINGS

The invention is described with reference to the following drawings, in which:
Figure 1 (a), (b) and (c) are atomic force microscopy (AFM) images taken at a 2µm scale, a 500nm scale and a 100nm scale respectively with an atomic force microscope in tapping mode on a section cut from the interior of a particle using microtoming and embedded in a standard resin. The particle was made according to the process defined herein.
Figure 2 is a schematic representation of an apparatus suitable for the production of particles of the present invention.
Figure 3 is a schematic representation of the nozzle used in the apparatus shown in Fig. 2.
Figure 4a is a size distribution chart for encapsulated F&DC Yellow 5 particles made according to Example 1.
Figure 4b is a size distribution chart for encapsulated Acid Red No.27 particles made according to Example 2.
Figure 4c is a size distribution chart for encapsulated F&DC Blue No.1 particles made according to Example 3.
Figure 5 is the calibration for ion-exchanged Tartrazine in water at 423 nm.
Figure 6 shows the tartrazine leakage obtained in Example 4B.

### DETAILED DESCRIPTION OF THE INVENTION

The dimensions and values disclosed herein are not to be understood to be strictly limited to the exact numerical values recited. Instead, unless otherwise stated, each dimension is intended to mean both the recited value and a functionally equivalent range surrounding that value. For example, a dimension disclosed as "40nm" is intended to mean "about 40nm".

In the context of the present invention, the term "encapsulation" is understood to mean that the dye is fully surrounded or encased by an encapsulating agent and, thus, held securely within the particle. Leakage of less than 5 weight %, preferably less than 2 weight%, more preferably less than 1 weight% of the total amount of dye incorporated into the particle is achieved, as determined by the methodology described herein in Example 4.

Both the present particles and the encapsulating agent comprised within the particles of the present invention are amorphous. In the context of the invention, the term "amorphous" means that there is no long range crystallographic order in one, two or three dimensions at lengths from 0.1- 50nm, as determined in the following way by a combination of powder x-ray diffraction (XRD) on bulk samples and transmission electron microscopy (TEM) of representative portions of the same bulk sample:
(a) The presence of a broad peak in the x-ray diffractogram centered between 2 theta angles corresponding to d-spacings of 0.37-0.42 nm, with full width half-maximum (FWHM) of between 5-10 degrees 2 theta;
(b) The lack of sharp powder x-ray diffraction peaks correspondings to spacings of crystallographic planes separated by 0.37-0.42nm;
(c) The lack of mesocrystalline order (where respective highest order Bragg peaks fall in the range 2-50nm - typical of ordered mesostructured materials), as determined by TEM imaging of samples prepared by microtoming;
(d) The lack of a multiplicity of sharp peaks in the range of two theta angles corresponding to d-spacings to 0.1-50nm.

This definition excludes ordered mesoporous materials with pore sizes from 2-50nm arranged with translational crystallographic order, such as MCM-41, MCM-48 and SBA-15.

In the context of the present invention, the term "siliceous" takes its normal meaning known in the art. More specifically, a siliceous material is one of, relating to or containing silica or a silicate. Preferably, the encapsulating agent is silica *per se.* Optionally, however, a proportion of the silicon within the amorphous silica structure may be substituted with other elements such as boron, lead, titanium, tin, zirconium and/or aluminium. This substitution of the silica framework may be useful in adjusting the properties of the silica-based particles depending upon their specific applications. For example, addition of boron, lead, tin, zirconium and/or aluminium may result in a different refractive index.

In addition, depending upon the desired applications and/or effects of the particles, it may be desirable for them to additionally comprise one or more inorganic particles, such that they comprise not only the siliceous encapsulating agent and one or more dyes, but also discrete inorganic, preferably refractory, particles such as titanium dioxide, zinc oxide, aluminium oxide and mixtures thereof, within their structure. For instance, titanium oxide and zinc oxide may provide additional sunscreen benefits. Such additional particles preferably have a mass average particle size of less than about 1 µm, preferably less than 100nm.

The dye molecules are typically present within the particle in more than one area or "pocket". This beneficial maximises the dye to particle volume or weight ratio and, thus, maximises the amount of dye ultimately included in the desired end compositions, for example the cosmetic, health, beauty or detergent products or ink compositions, whilst minimising the overall proportion of dye particles within such compositions. Irrespective of the number of areas or "pockets" of dye within the particle, each is fully surrounded or encapsulated by the encapsulating agent and, thus, held securely therein. Therefore, within the structure of the particles themselves, the encapsulating agent may be thought of as a continuous phase or matrix, whereas the dye may be thought of as comprised within a discontinuous phase. It follows that an "encapsulating agent" is an agent, which may be used to achieve this effect.

In this respect, the encapsulating agent may also be considered to be polymeric in nature because it will tend to possess crosslinking within its structure. It is preferred that the particles of the invention have as high a degree of crosslinking as possible, such that the dye is most effectively retained within the resulting particle and cannot leach therefrom. The degree of crosslinking may be observed using standard techniques such as Fourier transform infrared spectroscopy (FTIR) or solid state nuclear magnetic resonance spectroscopy (solid state NMR). Ideally and as previously mentioned, leakage of less than 5 weight %, preferably less than 2 weight%, more preferably less than 1 weight% of the total amount of dye incorporated into the particle is achieved, as determined by the methodology described herein in Example 4.

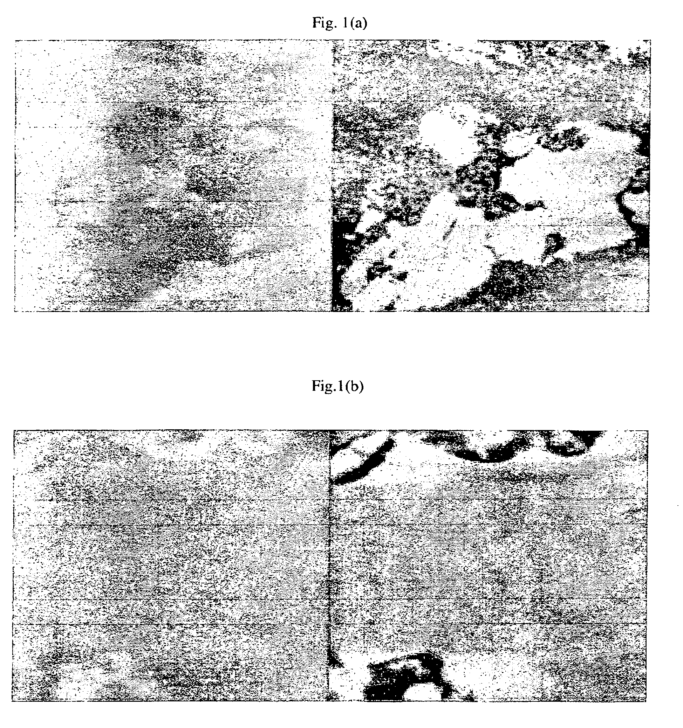

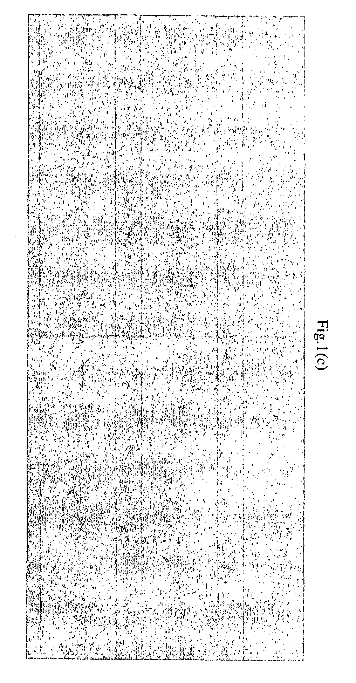

Additionally, the particles of the invention comprise a homogeneous distribution of the one or more dyes within the encapsulating agent. In the context of the present invention, this "homogeneous distribution" of the dye is understood to mean that the dye is homogeneously dispersed throughout the particle on a "molecular level". This means that the dye, typically present in one or more areas or "pockets", is not visible or discernible *via* microscopic techniques down to a range or magnification of 2nm. In other words, the particles of the invention appear as a homogeneous or single material at this level of microscopic magnification. This is clearly illustrated by the AFM images shown Figure 1, which were taken with an atomic force microscope in tapping mode on a section cut from the interior of a particle using microtoming and embedded within a resin. The particle of the invention investigated by this technique was that of Example 1 herein.

The AFM image on the left hand side of Figure 1 (a) - (c) shows the variation within the particle in terms of its height, whereas the AFM image shown on the right hand side of Figure 1 shows the variation in phase within the particle. Each of the images shows that the composition of the particle is essentially homogeneous over this area range and, indeed, down to a range of at least 2nm, ie. "at a molecular level". The phase image on the right hand side of Figure 1(a) - (c) essentially records an image based upon the differential hardness of the particle sample. From the phase image, it may in fact be concluded that the particle is uniformly textured almost to the resolution limit of the highest magnification image. Accordingly, it may be concluded that any encapsulating agent cages or pockets, within which the dye molecules are held, must therefore be in the range of less than approximately 2 nm in average diameter or size. Furthermore, the AFM images do not identify any regions of dye aggregates, which would appear softer and a different colour in the phase image. Therefore, the AFM results appear to show that the dye molecules are homogeneously distributed in pockets or cages within the silica network. '

Without wishing to be bound by theory, therefore, the compositional homogeneity of the particles, as shown particularly well by the AFM images in Figure 1 (a) - (c), appears to be key to the retention of the dye within the particles. This homogeneity has not been seen with particles of the prior art.

Turning now to the dyes included in the particles of the invention, a wide variety of dyes is suitable for this purpose. In the context of the present invention, the term "dye" refers to any dye or colorant, which is desired to be introduced into a particle and indefinitely retained within that particle. Examples of dyes which may be comprised within particles of the present invention include, but are not limited to, dyes or colorants conventionally used in the end application(s) of choice. For example, the suitability of dyes for use in applications such as cosmetic, health, personal care and detergent compositions is governed by organisations such as the Food and Drug Administration (FDA) in the USA and equivalent bodies in other countries. Typically, dyes suitable for use in the present invention may be cationic, anionic, neutral, amphoteric, zwitterionic or amphiphilic, with cationic dyes being preferred, as the positive charge on the dye molecule interacts with residual negative charge on the siliceous encapsulating agent to promote retention of the dye within the encapsulant. The dyes are typically selected from conventionally-known dye types such as natural dyes, ie. those derived from natural sources or synthetic equivalents thereof, azo dyes, indigoid dyes, triaryl-methane dyes, anthraquinone dyes, xanthine (xanthene) dyes, nitrosulphonate dyes, pyrene dyes, thiophene dyes, quinoline dyes and derivatives, lakes, composites or mixtures thereof, in particular those which have been approved for use by the FDA. Examples of suitable dyes are provided in the following tables (1 and 2), with their general dye types shown in brackets.

| **Table 1: Colour Additives batch-certified by the FDA** | | |
|---|---|---|
| **Standard Name** | **Chemical Structure** | **Colour Index Number (CI)** |
| FD&C Black No. 2 | Carbon black | 77266 |
| FD&C Orange No. 4 (monoazo) | 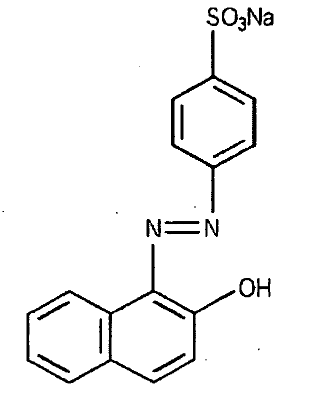 | 15510 |
| FD&C Orange No. 5 (xanthene-based) | 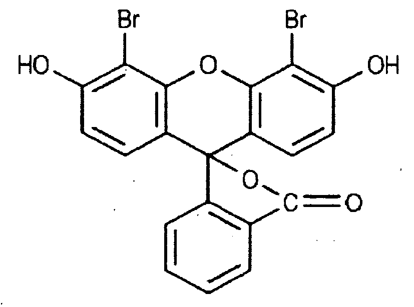 | 45370 |
| FD&C Orange No. 10 (xanthene-based) | 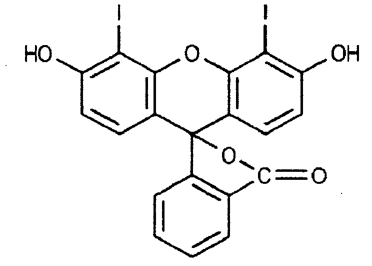 | 45425 |
| FD&C Orange No. 11 (Sodium salt of Orange No. 10; xanthene-based) | 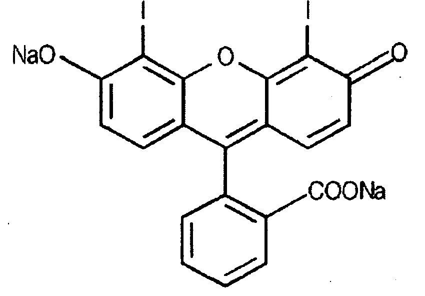 | 45425 |
| FD&C Blue No. 1 (triarylmethane; "Erioglaucine") | 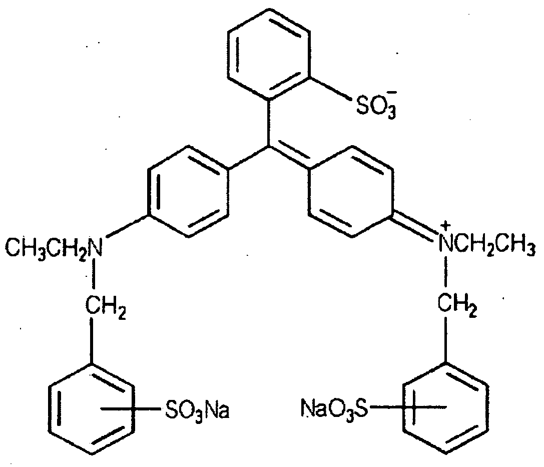 | 42090 |
| FD&C Blue No. 4 (triarylmethane) | 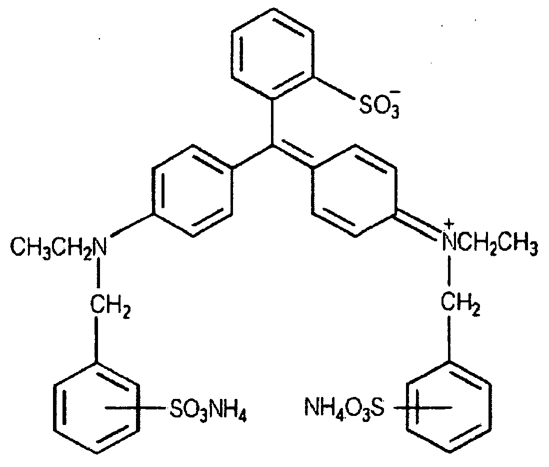 | 42090 |
| FD&C Brown No. 1 (diazo) | 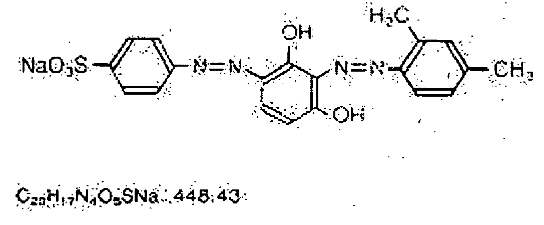 | 20170 |
| FD&C Violet No. 2 (anthracene dione-based; ie. anthraquinone based) | 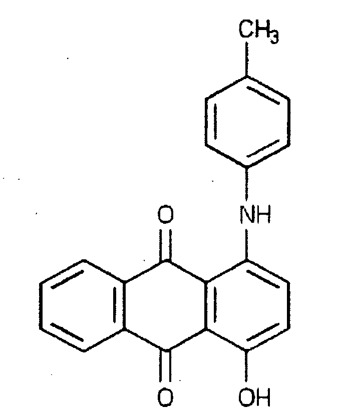 | 60725 |
| Ext. D&C Violet No. 2 (anthracene-based) | 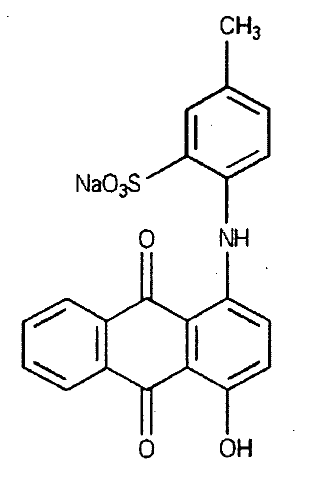 | 60730 |
| FD&C Green No. 3 (triarylmethane) | 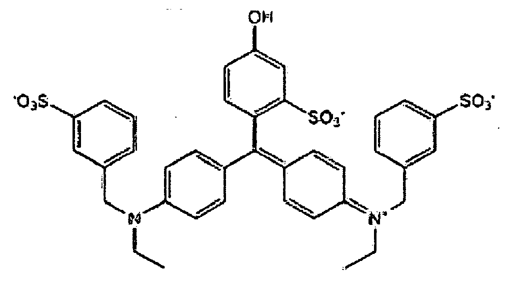 | 42053 |
| FD&C Green No. 5 (anthracene-based) | 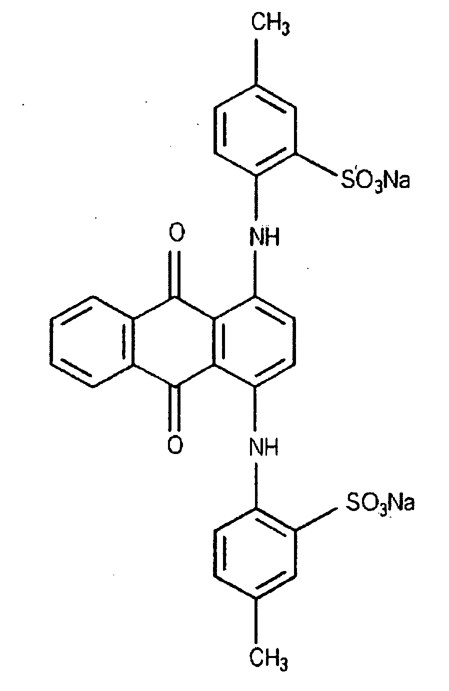 | 61570 |
| FD&C Green No. 6 (anthracene-based) | 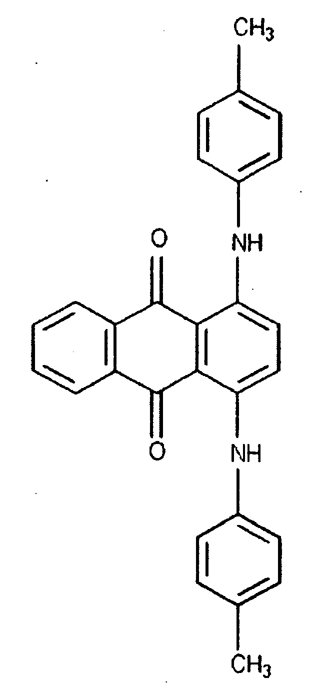 | 61565 |
| FD&C Green No. 8 (pyrene-based) | 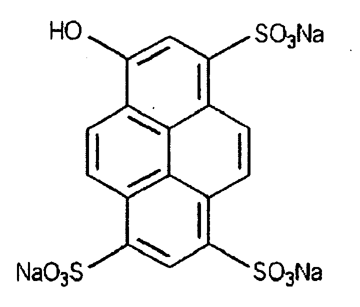 | 59040 |
| FD&C Red No. 2 (monoazo; "Amaranth") | 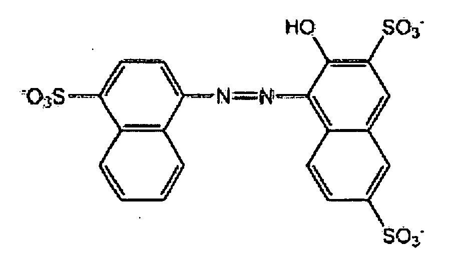 | 16185 |
| FD&C Red No. 4 (monoazo) | 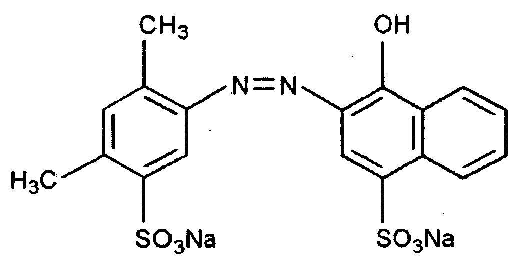 | 14700 |
| FD&C Red No. 6 (monoazo) | 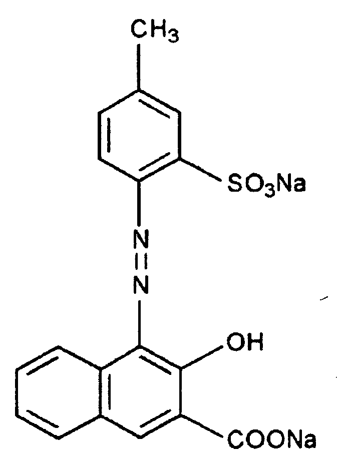 | 15850 |
| FD&C Red No. 7 (monoazo) | 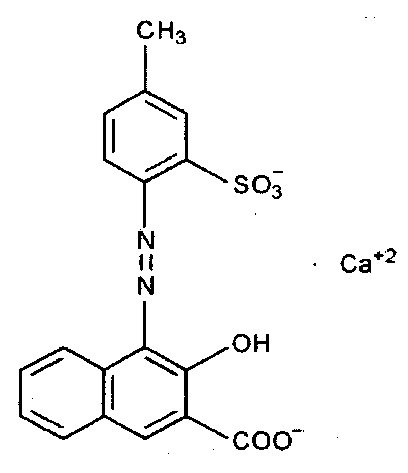 | 15850 |
| FD&C Red No. 17 (diazo) | 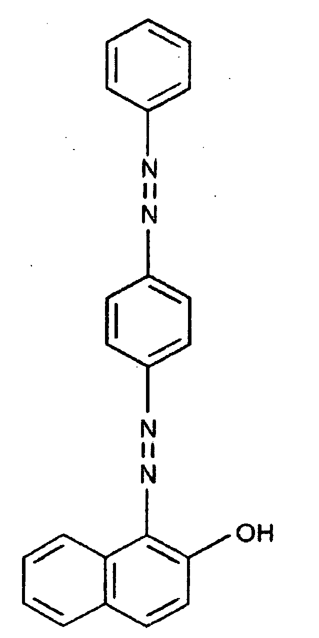 | 26100 |
| FD&C Red No. 21 (xanthene-based) | 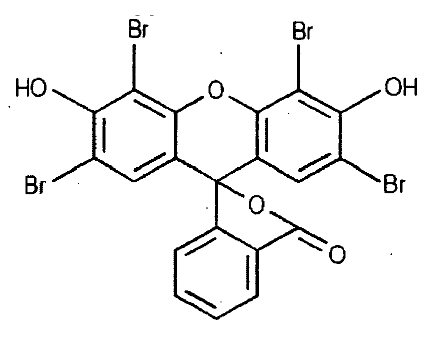 | 45380 |
| FD&C Red No. 22 (xanthene-based) | 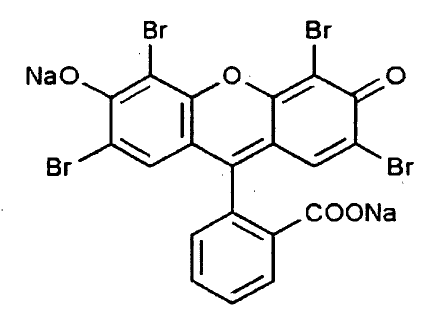 | 45380 |
| FD&C Red No. 27 (xanthene- based) | 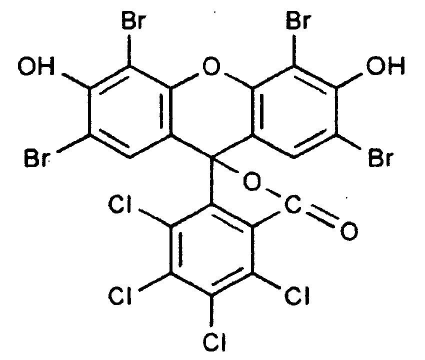 | 45410 |
| FD&C Red No. 28 (xanthene-based) | 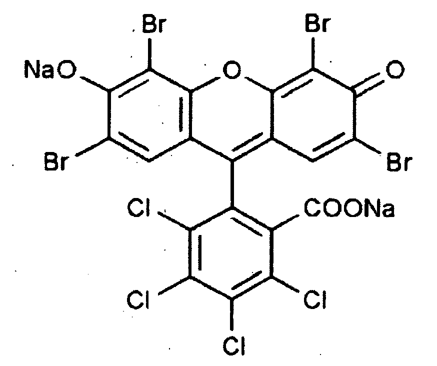 | 45410 |
| FD&C Red No. 30 (thiophene-based) | 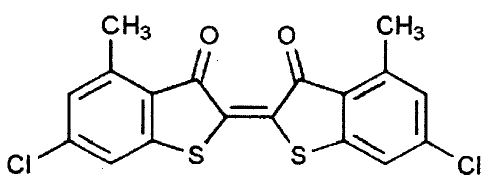 | 73360 |
| FD&C Red No. 31 (monoazo) | 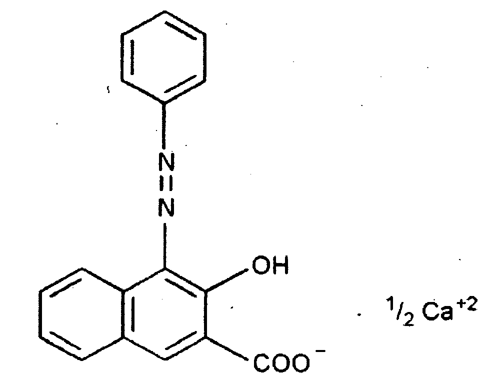 | 15800 |
| FD&C Red No. 33 (monoazo) | 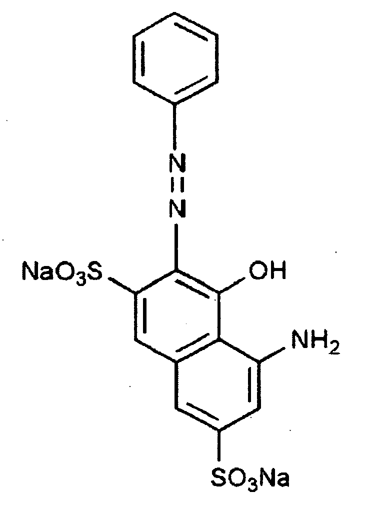 | 17200 |
| FD&C Red No. 34 (monoazo) | 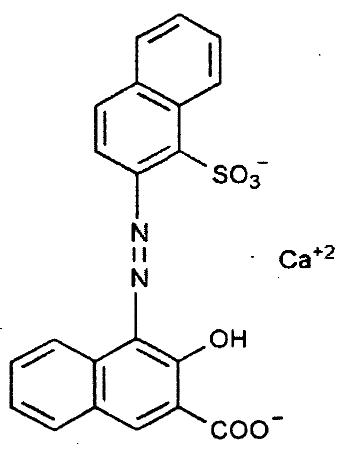 | 15880 |
| FD&C Red No. 40. (monoazo) | 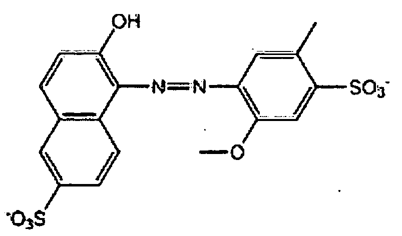 | 16035 |
| FD&C Yellow No. 5 (monoazo; "Tartrazine") | 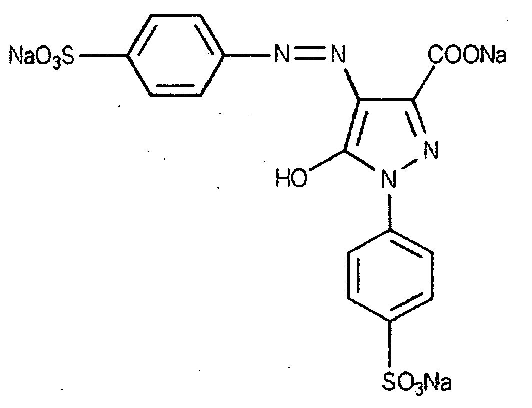 | 19140 |
| FD&C Yellow No. 6 (monoazo) | 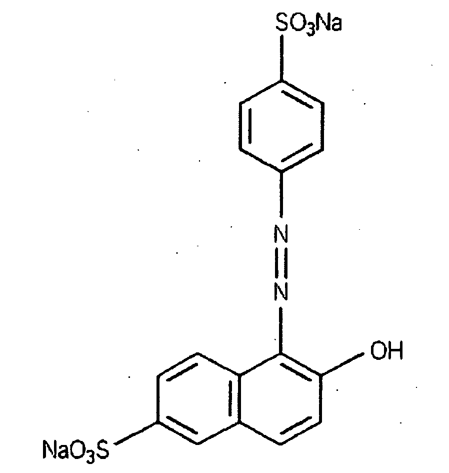 | 15985 |
| FD&C Yellow No. 7 (xanthene-based) | 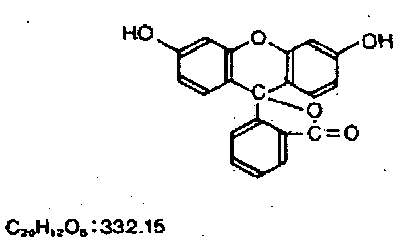 | 45350 |
| Ext. D&C Yellow No.7 (dinitroarylsulphonate) | 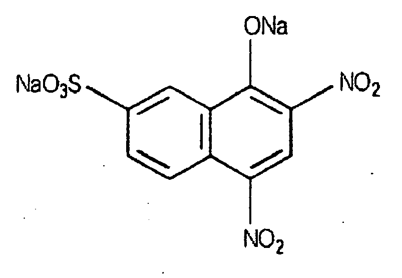 | 10316 |
| FD&C Yellow No. 8 (xanthene-based) | 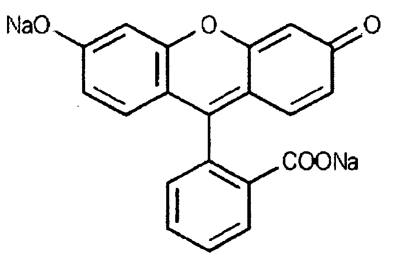 | 45350 |
| FD&C Yellow No. 10 (quinoline-based) | 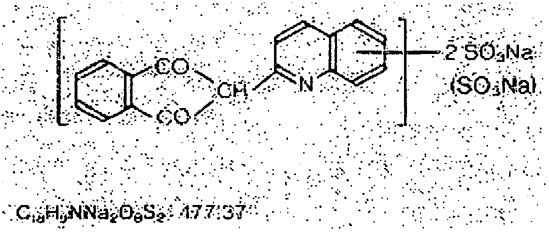 | 47005 |
| FD&C Yellow No. 11 (quinoline-based) | 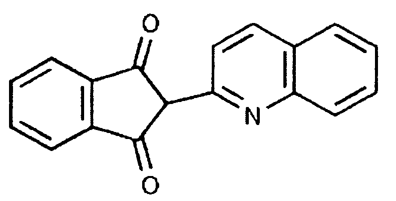 | 47000 |

| **Table 2: Natural Colour Additives which are Exempt from Batch Certification by the FDA** | | |
|---|---|---|
| **Name** | **Structure** | **CI** |
| Caramel | Not applicable (n//a) | |
| Cochineal | 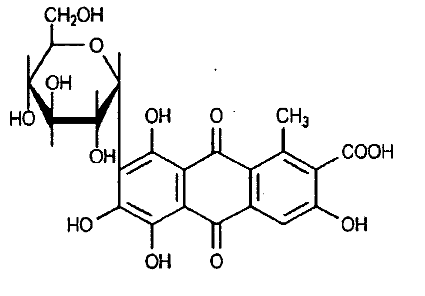 | 75470 |
| Beta carotene | 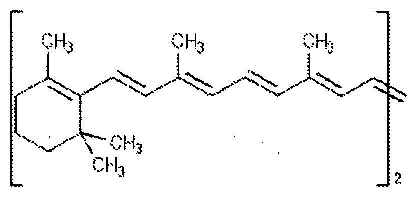 | 40800 or 75130 |
| Guanine | 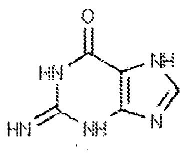 | 75170 |
| Henna | n/a | n/a |

The dye may be used in an unadulterated form or it may be adapted to improve its suitability to the present process, In particular, the effectiveness of some dyes containing anionic groups and a mono-valent alkali metal counter-ion, such as sodium, may be improved by ion-exchanging the metal ion with a mono-valent organic counter-ion such as ammonium ammonium.

Particularly preferred colorants or dyes include xanthene, triarylmethane, anthracene, and monoazo dyes.

The amount of dye included in the particles of the invention can be varied in accordance with the desired applications or effects of the particles, and may also depend upon the type(s) of dye chosen to be included within the particles. Within the particles of the invention, the proportion of dye(s) is from 3% to 20% hy weight of the particle. Preferably, the proportion of dye is in the range from 5% to 15% and more preferably from 8 to 12%, by weight of the particle. These ranges have been found to equate exactly to the percentages of the starting materials used to make the particles.

The particles of the invention have a volume average particle size which renders them useful in the end application of choice. For instance, if the particles are to be used in cosmetic or beauty formulations, it is desirable that they are not discernible to the naked eye. Thus, such particles will typically have an average size of less that about 70 µm. However, if the particles are destined for used in detergent or other formulations, they may have greater sizes, for example. For cosmetic applications, the average particle size is generally in the range of greater than 0 to 10 µm, preferably in the range of greater than 0 to 5 µm, more preferably from greater than 0 to less than 1 µm and even more preferably, is from 10nm to less than 1µm. The average particle size of the particles is measured using standard techniques of the art, such as light scattering *via* use of a Malvern Sizer 2000 apparatus or by scanning electron microscopy (SEM).

The particles of the invention may have any shape appropriate to the end use in question. Preferably, the particles according to the invention are spherical because such particles may have more predictable qualities, such as optical and rheological properties. Within a cosmetic application, spherical particles may also provide improved skin feel, since they may act as a lubricant by providing a ball-bearing type effect.

The particles of the invention achieve effective retention of dyes therein by an amorphous, siliceous encapsulating agent. The invention provides particles which possess good chemical and physical stability, colour fastness and tint strength as well as an acceptable environmental profile. A corollary of the low dye leakage is that the surface of the silica encapsulates according to the present invention has similar properties to silica per se, regardless of the dye(s) incorporated therein. Thus, the particles may be reliably and effectively incorporated into compositions for use in a wide variety of applications to provide colorants, which show negligible to no leakage from the compositions into which they are incorporated and which, at the same time, provide more robust coloration to the compositions than colorants of the art. Because of this, the particles of the invention may be effectively used to provide previously unattainable dye combinations, as the individual dyes are securely held in the inventive particles.

In addition, the dye particles of the invention may be formulated into bulk colorant compositions for convenient "drop-in" use in the desired end compositions. This is particularly advantageous as end compositions currently formulated typically require specific, tailored formulation of all their individual components, including their colorants, in order to provide the correctly formulated end composition. Thus, use of colorant particles of the present invention obviates the need for this repetitive, time-consuming and, therefore, uneconomic "custom" formulation by enabling the formulation of bulk end-product compositions which may then be coloured as desired using bulk colorant compositions comprising pre-determined proportions of dye particles made by the present invention.

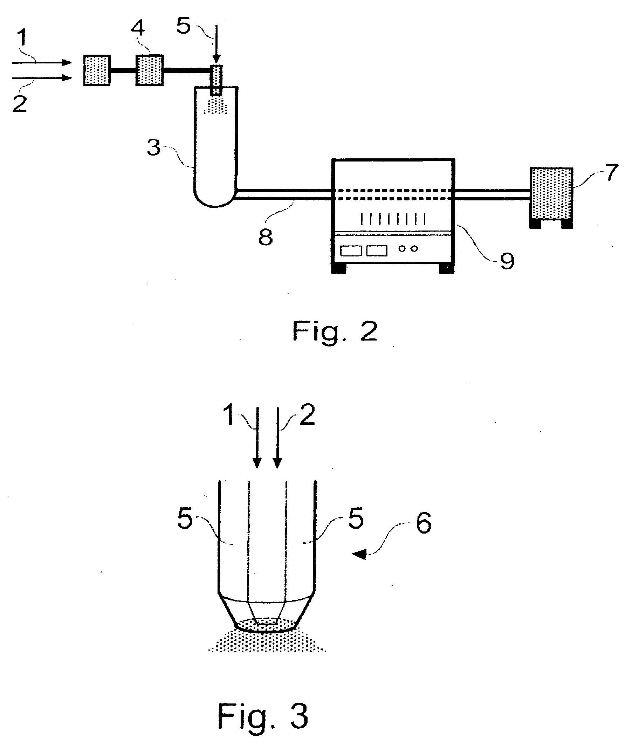

The particles of the invention may be made by any suitable known process, but are preferably made by an aerosol method. A suitable aerosol procedure is described with reference to Figure 2. In more detail, the encapsulating agent (1) and dye (2) are introduced in liquid form into a spray chamber (3), generally via means of a pump (4), together with a carrier gas (5) which is typically an inert gas such as nitrogen, or air dried by conventional methods for example. The liquid forms of the dye and encapsulating agent may be solutions, suspensions or dispersions, and are preferably both solutions. The liquid form of the encapsulating agent typically comprises at least one source or precursor of the siliceous encapsulating agent *per se* which, during the aerosol process, ultimately provides the desired siliceous encapsulating agent. The source of encapsulating agent may be considered to be a pre-polymer as, during aerosolisation, it will polymerise or crosslink to form the desired siliceous encapsulating agent. Preferably the siliceous precursor is organic. Suitable sources or precursors which may be used in the aerosol process to form particles of the invention include all those conventionally used in the art to form silica, silicates and zeolites, for example. Specific examples of useful silica precursors include tetramethylorthosilicate (TMOS), tetraethylorthosilicate (TEOS), tetrapropylorthosilicate (TPOS), tetraisopropylorthosilicate (TiPOS), tetrabutylorthosilicate (TBOS), silicic acid which may for example be modified with cations such as sodium or ammonium so that it is provided in the form of sodium silicate (also known as waterglass) or ammonium silicate. TEOS is a particularly preferred source of silica from a safety perspective, because the by-product of the process is ethanol (not methanol, as is the case for TMOS).

As is easily determinable and generally known by a skilled person, approximately one third of the weight of precursor is transformer into particles. For example, silica (SiO₂) has a molecular weight of 60g/mole and TEOS has a molecular weight of 208g/mole, so the weight of silica produced is 60/208 or 0.29 times the amount of TEOS. For TMOS the value is 0.39 (the molecular weight of TMOS is 152g/mole).

In addition, the amount of dye encapsulated within the particles of the invention is easily calculable by a skilled person. For example, if one wants a 10% dye loading, then, bearing in mind how much precursor one needs, as discussed above, it is a straightforward matter to calculate the amount of dye needed (a precursor solution of 10.4g of TEOS and 0.33g of dye, for example, yields silica particles comprising 90% silica (3g silica) and 10% dye (0.33g dye)).

The solvent used in the present invention will depend upon the hydrophobicity of the starting material. TEOS, TMOS and TPOS are hydrophobic and are therefore generally solubilised in an essentially non-aqueous material, for example as an alcoholic solution such as a solution in ethanol, methanol, n-propanol, iso-propanol and/or a butanol, ie. n-butanol, 2-butanol, iso-butanol or tert-butanol. Alternatively, a solution in acetone or one or more other conventional solvents, for instance, may also be employed. Silicic acid and the silicates are hydrophilic so may be dissolved in hydrophilic solvents such as water. The amounts of solvent used are readily determinable by a skilled person - the lower limit is, in practice, determined by the solubility parameters of the starting material and the upper limit is a practical one - the more solvent one uses, the smaller the final particles and the smaller the production capacity.

Although the solvent may be non-aqueous, some water is, nevertheless, necessary in order to hydrolyse the precursor, such as TEOS, to silicic acid prior to aerosolisation. Hydrolysis prior to aerosolisation is important to minimise the number of pores in then resulting particles, thereby minimising leakage of encapsulated dye. Typically, it is preferred that the aqueous portion be an acidic solution. The pH will be more than 1 and less than 7 and is advantageously approximately 2, as this is at or near the iso-electric point of silica itself. The pH of the precursor liquid form may be adjusted as desired using techniques conventionally used in the art, for example by addition of acid. A preferred acid used for this purpose is hydrochloric acid. Water of the requisite pH may be introduced as a solvent for the silica precursor or as a solvent for the dye, as discussed below.

The dyes incorporated into particles of the invention are provided to the aerosol process in acidic, basic or neutral liquid form. Preferably the one or more dyes is provided in the form of a solution in one or more solvents conventionally used in this field, preferably water or an alcohol such as methanol, ethanol, n-propanol, iso-propanol or a butanol (as previously defined), and particularly preferably ethanol or water. Highly preferably, the dye is provided in the form of an aqueous solution, and conveniently as an aqueous ethanolic solution. It may be necessary to aid dissolution of the active in the chosen solvent by providing it in the form of a salt, for instance those formed with commonly-used cations such as sodium, ammonium or potassium, or by adjusting the pH of the mixture of dye and solvent, again by conventional methods as previously described.

As mentioned above, it is desirable that, in creating the aerosol to form the particles of the invention, at least one of the liquid forms of encapsulating agent and dye should be aqueous. It is usually preferred that the dye be provided in aqueous liquid form, whereas the siliceous precursor is typically provided in non-aqueous form. The presence of water in the reaction medium aids pre-hydrolysis of the silica precursor which, in turn, aids subsequent polymerisation of the precursor to form the desired encapsulating agent comprising a low number of pores. The liquid forms of the dye and precursor are preferably mixed together prior to entry into the aerosol chamber for this purpose.

In addition and as mentioned above, if the siliceous encapsulating agent incorporates other inorganic materials in its structure in addition to silica, these may also be provided to the aerosol process in liquid forms of conventional sources of such materials. If it is desired to include titanium dioxide, for example, it may be appropriate to include a solution tetraethoxytitanate dissolved in an appropriate solvent, such as ethanol.

A suitable aerosol procedure is described with reference to Figure 2 in which the encapsulating agent (1) and dye (2) are mixed, then introduced in liquid form into a spray chamber (3), generally *via* means of a pump (4), together with a carrier gas (5) which is typically an inert gas such as nitrogen, or air dried by conventional methods for example.

Typically a spray nozzle (6) such as that shown in Figures 2 and 3 is used in the aerosol process, whereby the dye (2) and agent (1) are introduced through a central tube and the carrier gas (5) is introduced through an outer tube of the nozzle. This type of nozzle is conventionally known as a "two-flow spray nozzle", however, other nozzle types commonly used in creating aerosols may also be employed. A two flow nozzle is preferred as the carrier gas flow cuts across or dissects the central flow of dye and encapsulating agent, thus facilitating more effective formation of spray droplets comprising the encapsulating agent and dye. Whilst the apparatus shown in Figures 2 and 3 illustrates a downwardly-spraying nozzle, it will be appreciated that all conventional types of aerosol apparatus including upwardly spraying apparatus may be conveniently used in the aerosol process. Indeed, so-called "spray up" systems may be preferred where it is desirable to fractionate particles of different sizes directly from the spray chamber, for instance.

The droplets formed in the spray chamber (3) are typically held in the chamber for a residence time in the range of greater than 0 up to about three minutes. Residence time may affect the porosity and, to a limited extent, the size of the resulting particles. For instance, for an average particle size of approximately 3-5 µm and a minimum porosity, a residence time of approximately 10 seconds may be conveniently employed. When present in the spray chamber, the encapsulating agent undergoes crosslinking within itself, thus forming droplets of a secure cage-like structure or network within which the dye is securely held. In addition, of course, the solvents evaporate. Typically the particles according to the invention have a diameter which is half that of the droplets sprayed into the spray chamber (3).

The droplets are then removed from the spray chamber in a conventional manner for instance via means of a pressure differential created by a pump (7) located at the end of a tube (8), into which the droplets pass from the spray chamber. Generally, the tube (8) into which the droplets pass is heated to a temperature which will effect drying of the particles for instance via means of a heater (9). Typically, a temperature in the range of approximately 150-250 °C is employed. Heating of the droplets in this way promotes condensation and, thus, further crosslinking of the siliceous encapsulating agent, preferably ultimately resulting in the formation of substantially fully crosslinked polymer-encapsulated dye particles.

The particles made in the aerosol process are typically dried by any means conventionally known in the art, such as a heater, either before or after their recovery from the aerosol apparatus which is, again, achieved in a conventional manner.

Optionally, the particles may undergo a subsequent washing process, if so desired, in order to ensure that all of the dye is securely encapsulated within the particles and that none remains at the surface of the particles following the process of the invention, for example. Conventional washing agents or solvents such as water, alcohols or acetone may be used for this purpose, the choice of washing agent typically being dependent upon the solubility characteristics of the relevant dye(s).

The conditions under which particles of the invention are produced by the aerosol process are not critical. Accordingly, aerosolisation may be performed under temperature, pressure and other conditions as desired by the skilled person in this technical field. Typically and conveniently, however, aerosolisation is performed under ambient temperature and pressure conditions, ie. at room temperature of approximately 18-25°C, and at a pressure of approximately atmospheric pressure. However, it will be appreciated that lower or higher temperatures and pressures may be employed as desired. In addition, it is not essential to exclude humidity from the aerosol apparatus. As such, the relative humidity (RH) within the aerosol apparatus does not need to be monitored but, under ambient conditions, is typically less than 50 %, as measured by conventional techniques.

Particles according to the present invention have a specific surface area of 0.1m²/g to 2m²/g, preferably 0.5m²/g to 5m²/g, more preferably 0.5m²/g to 3.5m²/g. In addition, particles according to the present invention have a specific internal pore volume of 0.001 to 0.03 cm³/g, preferably 0.001m³/g to 0.011cm³/g. Surface areas and pore volumes are determined using nitrogen porosimetry using nitrogen at a temperature of -196°C or 77K. The samples are evacuated at 120-150°C for at least 4-6 hours to remove adsorbed water from the pores, and sample sizes are preferred to be around 0.5g. Otherwise standard procedures for collecting high quality N₂ isotherm data should be followed. The pore volumes are cumulative pore volumes for internal pores less than 50 nm in diameter and are determined using the "Barret-Joiner-Halenda" method.

### Examples

The present invention will now be described in more detail with reference to the following nonlimiting example(s):

### Example 1: Preparation of Silica loaded with Tartrazine (FD&C Yellow No.5)

As a first step to synthesising sodium tartrazine-containing silica, the dye (commercially available from Sigma as T0388-100G (CAS# 1934-21-0) was ion-exchanged using a column with ion-exchanging resin (type Dowex 50Wx8 commercially available Dow. Chemical Comp., Michigan, USA. This was necessary because the use of commercial Tartrazine induces flocculation of the tetraethylorthosilicate/ethanol/hydrochloric acid (TEOS/EtOH/HCl) encapsulating agent mixture.

### Column Preparation

The column was loaded with 317 g Dowex 50Wx8 to obtain a 400ml bed volume.
Step 1 -Washing: to remove residual sodium cations, the column was eluted with 2.51 deionized water over 5 to 10 minutes at pH 6.
Step 2 - Reconditioning: to remove bound sodium cations the column was washed through with four batches of 400 ml 7% HCl. The contact time of HCl on the column was 45 minutes.
Step 3 - Washing: as for Step 1.
Step 4 - Charging: as for Step 2, but it was performed using 7% ammonium chloride (NH₄Cl) instead of HCl.
Step 5 - Washing: as for Step 1, the purpose being to remove excess ammonium cations.

### Ion Exchange of Dye

A 10% dye solution of sodium tartrazine was prepared in an acidic solution of water and ethanol and was eluted through the column. This solution was used to produce Tartrazine-containing silica in the subsequent procedure.

### Spraying of silica with Yellow Dye

Two batches of *colou*red silica were each prepared using 10.4 g TEOS, 5.4 g of HCl with a pH of around 1.25 and 12.0 g of ethanol. The components were mixed together and the mixture was left stirring for 30 minutes. Theoretically, such a mixture should give 3 g of silica after aerosolisation. The calculation of the amount ofTartrazine solution to add was based on this theoretical amount of silica. The ion-exchanged Tartrazine solution was then mixed with 4 g of ethanol.

The two resulting mixtures were then blended together, the pH was adjusted to pH 2.0 using 1M HCl and the blend was left under stirring for a further 10 minutes. The blend was then aerosolized and spray-dried as follows:

The starting solution blend is pumped at a constant rate of 3ml/minute using a peristaltic pump to the centre flow outlet of a coaxial two-flow spray nozzle of a spray tower. At the same time, compressed air is pumped at 20litres/minute (at STP) to outer annular outlet located coaxially around the centre flow outlet. The centre flow outlet diameter is 1mm; the outer diameter is 1.5mm. The spraying was such that a turbulent mixture was propelled into the spray chamber, which was retained at ambient temperature. Afterwards, the mixture was heated to 220°C to induce cross-linking and drying of the particles.

### Washing of Particles

The particles were washed with de-ionized water, at a rate of 200 ml water per 1 g of particles as follows: 5 g of particles were placed into a plastic bottle and 1000 ml of water were added. The mixture was left under stirring for 5 minutes and it was then centrifuged for 10 minutes at 3500 rpm. The sediment was then separated from the supernatant fluid.

### Separation of Small Particles

The sediment from the centrifuged mixture was mixed with 1000 ml water in a beaker and left to settle for two days. The resulting supernatant fluid was then pumped into another beaker using a roll pump. Once the supernatant had been pumped into the other beaker, it was centrifuged at 3500 rpm for 20 minutes and the resulting sediment was separated using a standard separation technique from the liquid. The resulting particles were then dried in an oven at 50°C.

### Particle Size Measurements

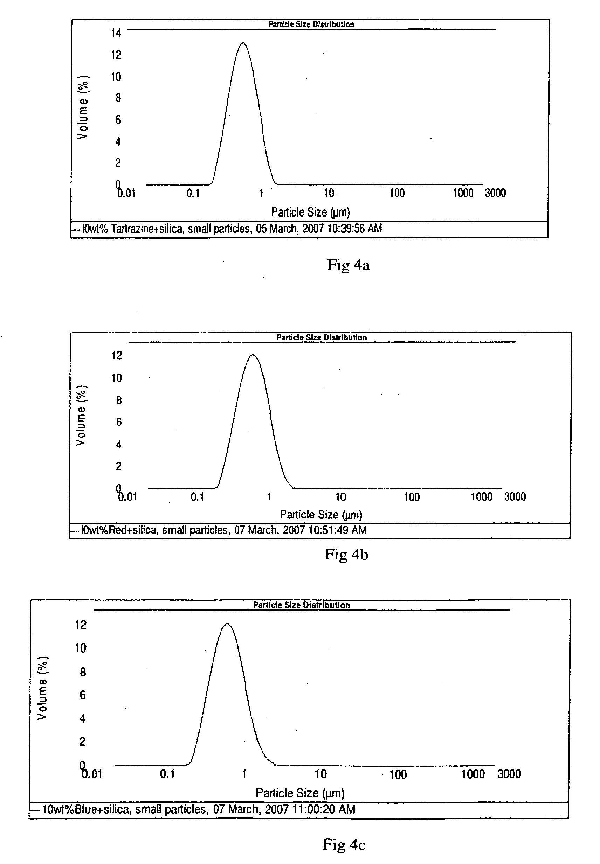

The size of the resulting particles was measured using a Malvern Master Sizer 2000 apparatus, which measures particle size via light scattering The particle size distribution is shown in the Figure 4a, in which

| | |
|---|---|
| d(0.1): | 0.308µm (10% of the particles have a size lower than the volume averaged value given) |
| d(0.5): | 0.539µm (50% of the particles have a size lower than the volume averaged value given) |
| d(0.9): | 0.953 µm (90% of the particles have a size lower than the volume averaged value given) |

### Example 2: Preparation of Silica loaded with Amaranth (Acid Red No.27)

This red dye is soluble in water but it is not soluble in ethanol. It was not necessary to ionexchange the aqueous solution (as was done for Tartrazine in Example 1), however, as it did not flocculate when it was blended with the TEOS mixture. Also, even though the dye was insoluble in ethanol, it was still possible to use it directly by increasing the water proportion in the aerosol mixture.

### Sample Preparation

Two batches of coloured silica were prepared using 10.4 g TEOS, 5.4g of HCl of pH 2 and 12.0 g of ethanol. The components were mixed together and the mixture was left stirring for 30 minutes. Theoretically, the mixture would give 3 g of silica after the aerosolisation. Thus, the calculation of the amount of Amaranth solution (obtained from Sigma has catalogue number A1016-100G (CAS#915-67-3)) to add was based on this amount of silica. This was as follows: 0.3 g of Amaranth powder plus 10.0 g of HCl (pH 2). The two mixtures were mixed together and left to stir for 10 minutes. The mixture was subsequently spray dried, heated, washed, the particles separated and size measured as in example 1. The particle size results are shown in Fig.4b, and:

| | |
|---|---|
| d(0.1): | 0.322µm |
| d(0.5): | 0.592µm |
| d(0.9): | 1.130µm |

### Example 3: Preparation of Silica loaded with Erioglaucine (FD & C Blue No. 1)

This blue dye is soluble in water and ethanol and it was not necessary to ion exchange the solution.

### Sample Preparation

Two hatches of coloured silica were prepared each using 10.4 g TEOS, 5.4 g HCl (pH2) and 8.0 g of ethanol. The components were mixed together and the mixture was left under stirring for 30 minutes. Theoretically, the mixture would give 3g of silica after the aerosolisation. Thus, the calculation of the amount of Erioglaucine solution to add was based on this amount of silica. This was as follows: 0.3 g of Erioglaucine powder (obtained from Sigma/Aldrich, catalogue# 861146-25G (CAS#3844-45-9)) plus 2.0 g of HCl (pH 2) in 3 g ethanol. The two mixtures were mixed together and left to stir for 10minutes. The mixture was subsequently spray dried, heated, washed, the particles separated and size measured as in example 1. The particle size results are shown in Fig.4c, and:

| | |
|---|---|
| d(0.1): | 0.327 µm |
| d(0.5): | 0.59 µm |
| d(0.9): | 1.130 µm |

### Example 4: Dye Release/Leakage Experiments

### Example 4A

For the products of each of Examples 1, 2 and 3, 0.2 g of the particles loaded with dye were placed into a centrifuge tube and 10 ml of a water/propanol (1:1) mixture was added. The tube was shaken for two minutes and centrifuged. The supernatant fluid was separated from the sediment and collected in a bottle. This operation was repeated five times. The supernatant fluid from all five extractions was mixed and analysed using a UV spectrometer The results are provided in the following table (3).

| **Table 3** | | | |
|---|---|---|---|
| Wash number | Release Yellow, wt% | Release Red, wt% | Release Blue, wt%. |
| 1 | 0.25 | 0.36 | 0.1299 |
| 2 | 0 | 0.027 | 0.0196 |
| 3 | 0 | 0 | 0.0091 |
| 4 | 0 | 0 | 0 |
| 5 | 0 | 0 | 0 |
| Sum over five washes | 0.25 | 0.387 | 0.1586 |

The results show that the release of the dyes into the water/propanol mixture was extremely low.

### Example 4B

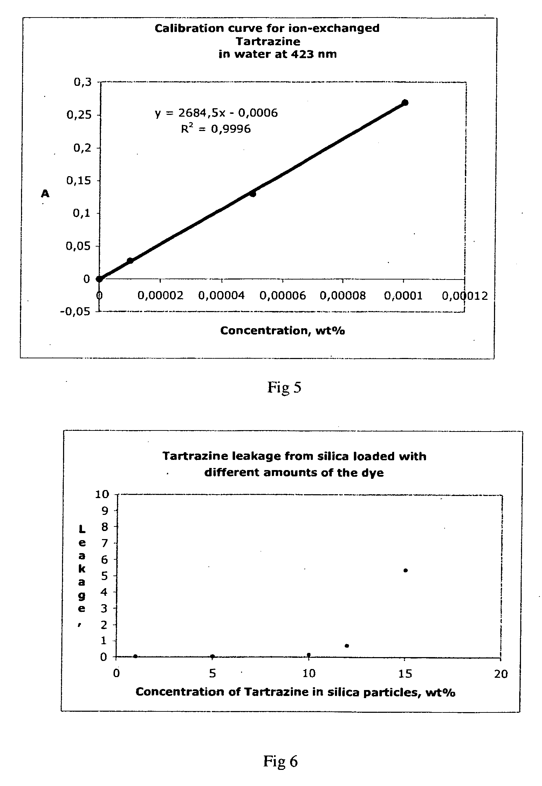

The leakage of Tartrazine from silica loaded with different amounts of dye was investigated as follows. In turn, I g of particles loaded with 1%, 5%, 10%, 12% and 15% respectively of Tartrazine was placed into a bottle and 100 g of water was added. The mixture was left under stirring for 3 hours, after which time, a 5 ml portion was extracted from every bottle using a syringe. This portion was filtered with a membrane filter (0.45 µm) and analyzed in an UV-VIS spectrophotometer. The leakage in wt% was calculated for every sample with help of the calibration curve presented as Figure 5, which shows the calibration for ion-exchanged Tartrazine in water at 423 nm.

The results are presented in the following table (4).

| **Table 4** | |
|---|---|
| **Tartrazine Concentration (wt%)** | **Leakage (wt%)** |
| 1 | 0.044 |
| 5 | 0.0528 |
| 10 | 0.155 |
| 12 | 0.727 |
| 15 | 5.37 |

These results are presented in Figure 6, and show that dye leakage, in the case of Tartrazine-loaded particles, substantially increases at greater than 12 wt% Tartrazine concentration within the particles.

## Claims

1. An amorphous particle comprising a homogeneous distribution of one or more dyes encapsulated by an amorphous, siliceous encapsulating agent, wherein the amorphous particle comprises from 3% to 20% dye, by weight of the particle, and wherein the dye is homogeneously dispersed throughout the particle.

2. A particle according to claim 1, wherein the encapsulating agent is silica.

3. A particle according to claim 1, wherein the siliceous encapsulating agent comprises ones or more of titanium, tin, zirconium and aluminium.

4. A particle according to any preceding claim, wherein the one or more dyes is cationic.

5. A particle according to any preceding claim, wherein the or each dye is xanthene, triarylmethane, anthracene, monoazo dye or a mixture thereof.

6. A particle according to any one of the preceding claims, comprising from 5% to 15%, preferably from 8% to 12% of the one or more dyes, by weight of the particle.

7. A particle according to any preceding claim, additionally comprising one or more types of inorganic particulate material.

8. A particle according to any preceding claim, having a specific surface area of 0.5m²/g to 5m²/g, preferably 0.5m²/g to 3.5m²/g.

9. A particle according to any preceding claim having a specific internal pore volume of 0.001 to 0.03 cm³/g, preferably 0.001m³/g to 0.011cm³/g.

10. A particle according to any preceding claim, which is spherical.

11. A particle according to any preceding claim, made by aerosolising an acidic precursor solution, then heating the aerosolised droplets to cross-link the siliceous encapsulating agent.

12. A plurality of particles according to any preceding claim, the particles having an average particle size of greater than 0 to 10 µm, preferably greater than 0 to 5 µm, more preferably greater than 0 to less 1. µm.

13. A plurality of particles according to claim 7, the particles having an average particle size of from 10 nm to less than 1 µm.

## Patentansprüche

1. Amorphes Teilchen, das eine homogene Verteilung mindestens einen Farbstoffe umfasst, die von einem amorphen, siliciumhaltigen Ummantelungsmittel ummantelt sind, wobei das amorphe Teilchen, bezogen auf das Gewicht des Teilchens, 3 % bis 20 % Farbstoff umfasst, und wobei der Farbstoff homogen in dem gesamten Teilchen verteilt ist.

2. Teilchen nach Anspruch 1, wobei das Ummantelungsmittel Siliciumdioxid ist.

3. Teilchen nach Anspruch 1, wobei das siliciumhaltige Ummantelungsmittel eines oder mehrere der Elemente Titan, Zinn, Zirconium und Aluminium umfasst.

4. Teilchen nach einem der vorstehenden Ansprüche, wobei der mindestens eine Farbstoff kationisch ist.

5. Teilchen nach einem der vorstehenden Ansprüche, wobei der mindestens eine Farbstoff Xanthen-, Triarylmethan-, Anthracen- oder Monoazo-Farbstoff oder eine Mischung davon ist.

6. Teilchen nach einem der vorstehenden Ansprüche, das, bezogen auf das Gewicht des Teilchens, zu 5 % bis 15 %, vorzugsweise zu 8 % bis 12 % den mindestens einen Farbstoff umfasst.

7. Teilchen nach einem der vorstehenden Ansprüche, das zusätzlich mindestens eine Art von anorganischem teilchenförmigem Material aufweist.

8. Teilchen nach einem der vorstehenden Ansprüche mit einer spezifischen Oberfläche von 0,5 m²/g bis 5 m²/g, vorzugsweise 0,5 m²/g bis 3,5 m²/g.

9. Teilchen nach einem der vorstehenden Ansprüche mit einem spezifischen Poreninnenvolumen von 0,001 bis 0,03 cm³/g, vorzugsweise 0,001 m³/g bis 0,011 cm³/g.

10. Teilchen nach einem der vorstehenden Ansprüche, das kugelförmig ist.

11. Teilchen nach einem der vorstehenden Ansprüche, das durch Aerosolieren einer sauren Vorläuferlösung und anschließendes Erwärmen der aerosolierten Tröpfchen, um das siliciumhaltige Ummantelungsmittel zu vernetzen, hergestellt ist.

12. Vielzahl von Teilchen nach einem der vorstehenden Ansprüche, wobei die Teilchen eine durchschnittliche Teilchengröße von mehr als 0 bis 10 µm, vorzugsweise von mehr als 0 bis 5 µm, stärker bevorzugt von mehr als 0 bis weniger als 1 µm aufweisen.

13. Vielzahl von Teilchen nach Anspruch 7, wobei die Teilchen eine durchschnittliche Teilchengröße von 10 nm bis weniger als 1 µm aufweisen.

## Revendications

1. Particule amorphe comprenant une répartition homogène d'une ou plusieurs teintures encapsulées par un agent d'encapsulation siliceux amorphe, où la particule amorphe comprend de 3 % à 20 % de teinture, en poids de la particule, et dans laquelle la teinture est homogènement dispersée sur l'ensemble de la particule.

2. Particule selon la revendication 1, dans laquelle l'agent d'encapsulation est de la silice.

3. Particule selon la revendication 1, dans laquelle l'agent d'encapsulation siliceux comprend un ou plusieurs parmi le titane, l'étain, le zirconium et l'aluminium.

4. Particule selon l'une quelconque des revendications précédentes, dans laquelle les une ou plusieurs teintures sont cationiques.

5. Particule selon l'une quelconque des revendications précédentes, dans laquelle la ou chaque teinture est une teinture de xanthène, triarylméthane, anthracène, monoazo ou un mélange de celles-ci.

6. Particule selon l'une quelconque des revendications précédentes, comprenant de 5 % à 15 %, de préférence de 8 % à 12 % des une ou plusieurs teintures, en poids de la particule.

7. Particule selon l'une quelconque des revendications précédentes, comprenant en outre un ou plusieurs types de matériau particulaire inorganique.

8. Particule selon l'une quelconque des revendications précédentes, ayant une surface spécifique de 0,5 m²/g à 5 m²/g, de préférence 0,5 m²/g à 3,5 m²/g.

9. Particule selon l'une quelconque des revendications précédentes ayant un volume spécifique de pores internes de 0,001 à 0,03 cm³/g, de préférence 0,001 m³/g à 0,011 cm³/g.

10. Particule selon l'une quelconque des revendications précédentes, qui est sphérique.

11. Particule selon l'une quelconque des revendications précédentes, fabriquée par brumisation d'une solution de précurseur acide, puis chauffage des gouttelettes en suspension pour réticuler l'agent d'encapsulation siliceux.

12. Pluralité de particules selon l'une quelconque des revendications précédentes, les particules ayant une taille moyenne de particules supérieure à 0 à 10 µm, de préférence supérieure à 0 à 5 µm, plus préférablement supérieure à 0 jusqu'à moins de 1 µm.

13. Pluralité de particules selon la revendication 7, les particules ayant une taille moyenne des particules allant de 10 nm jusqu'à moins de 1 µm.
